# EUROPEAN PATENT APPLICATION

(11) **EP 4 550 340 A1**
(43) Date of publication of application: **07.05.2025**
(21) Application number: 23207245.4
(22) Date of filing: 01.11.2023
(51) Int. Cl.: G16H 10/20, G16H 10/60, G06F 21/64

(54) **BLOCKCHAIN DATA STORAGE SYSTEM AND METHOD**

(71) Applicant: BIOTRONIK SE & Co. KG, 12359 Berlin (DE)
(72) Inventor: Lang, Volker, 12161 Berlin (DE); Diem, Björn Henrik, 14197 Berlin (DE); Wist, Dominic, 10318 Berlin (DE); Schulze-Luckow, Sebastian, 10715 Berlin (DE); Becker, Frank, 10409 Berlin (DE); Kraetschmer, Hannes, West Linn, 97068 (US); Parish, Patrick L., Portland, 97219 (US); Lim, Koeun, Tucson, 85750 (US)
(74) Representative: Biotronik Corporate Services SE

(57) **Abstract**

The invention relates to a blockchain data storage system (1) for clinical trial data (D) comprising a communication interface (10) configured to receive clinical trial data (D) and a plurality of nodes (12, 14, 16) configured to store the clinical trial data (D) related to events (E) along the course of at least one clinical trial. Furthermore, the invention relates to a computer-implemented method for storing clinical trial data (D) on a blockchain data storage system (1) comprising the steps of receiving clinical trial data (D) by a communication interface (10) and storing the clinical trial data (D) related to events (E) along the course of at least one clinical trial on a plurality of nodes (12, 14, 16) of the blockchain data storage system (1).

## Description

The invention relates to a blockchain data storage system for clinical trial data.

Furthermore, the invention relates to a computer-implemented method for storing clinical trial data on a blockchain data storage system.

Clinical studies are regularly conducted by medical technology companies. The aim of the studies is to determine the compatibility of drugs and/or medical devices, i.e., the subject of the study, with humans. The results of clinical studies therefore have a particular medical and commercial benefit in the development of the object of investigation. If the results of a clinical study are positive, a possible approval of the object of investigation for a specific market as a medical device comes closer.

A comprehensible and secure follow-up of the circumstances, contractor, participants, methods and results is therefore of great importance. This is especially true for the patient's representative, the national regulatory authority. It decides by admission of a drug and/or medical device about the safety of the patient. Conversely, contractors benefit from clinical trials that are documented in a comprehensible and secure manner, since they bear the economic risk of the clinical trial. Secure tracking of the clinical trials furthermore reduces this risk by making falsification more difficult.

US 2017/0076049 A1 discloses a system for recording and sharing medical record data or data relating to a medical clinical trial and patient data associated therewith in a manner that creates and preserves an electronic record of stored original data so that a verifiable electronic source document is created and preserved.

The system comprises a first electronic processing device disposed in communication with a software package that is capable of variably and selectively defining a plurality of access and permissions managers selected from medical care and clinical trials principals in an access and permissions manager selection set comprising a physician, a licensed health care provider, a clinical trial investigator, a medical record administrator, a clinical trial data record administrator, a site management organization, a contract research organization, a clinical trial administrator, a clinical trial monitor, a regulatory agency, a clinical trial sponsor, and an institutional or ethical review board, each of whom is capable of variably and selectively assuming a plurality of roles, including the roles of the other access and permissions managers; defining a plurality of roles associated with said plurality of access and permissions managers; defining a plurality of access and permissions sets for said plurality of roles of the access and permissions managers; and associating the assignments of the plurality of roles to the plurality of access and permissions managers; variably and selectively defining the assignments of the plurality of roles to the access and permissions managers, and the plurality of access and permissions sets for the plurality of roles of the access and permissions managers.

Furthermore, the system comprises a database, wherein said database further comprises data tables that contain fields defined for receiving and storing medical record data or medical clinical trial data, and patient data associated therewith.

The system moreover comprises a second electronic processing device disposed in communication with said first electronic processing device, said software package and said database, said second electronic processing device further comprising a graphical user interface that displays a plurality of defined data fields associated with corresponding data fields defined within the database, wherein access to said plurality of defined data fields displayed in said graphical user interface, and therefore to corresponding data fields defined within said database, is controlled by said plurality of access and permissions managers variably and selectively providing and restricting access to said plurality of defined data fields in response to the setting of associated access and password restrictions controlled by said plurality of access and permissions managers, and wherein said system further comprises a means for optional integration with one or more other data storage or management systems, said means for optional integration further comprising means for transmitting data to and receiving data from said one or more other data storage or management systems.

WO 2021/237345 A1 discloses a method to manage exposure of confidential user information in a user data record to a third party, the method comprising providing an electronic record system managed by a server arrangement, the server arrangement being configured to interact with a user device associated with the user, implementing with the server arrangement a data privacy configurator including a GUI allowing the user at the user device to specify data access parameters, the data access parameters distinguishing between first data in the user data record that the user is willing to expose to a third party from second data in the user data record that the user is not willing to expose to the third party, in response to a request received at the server arrangement to communicate user data from the user data record to the third party, processing the user data record according to the data access parameters to allow the third party to access the first data while precluding the third party to access the second data.

The object of the invention is thus to store the data generated during clinical trials in a traceable and immutable manner and to ensure access for the stakeholders in a suitable way.

This object is solved by a blockchain data storage system for clinical trial data having the features of claim 1.

Furthermore, the object is solved by a computer-implemented method for storing clinical trial data on a blockchain data storage system having the features of claim 14.

Further developments and advantageous embodiments are defined in the dependent claims.

The present invention provides a blockchain data storage system for clinical trial data comprising a communication interface configured to receive clinical trial data and a plurality of nodes configured to store the clinical trial data related to events along the course of at least one clinical trial.

The present invention further provides a computer-implemented method for storing clinical trial data on a blockchain data storage system comprising the steps of receiving clinical trial data by a communication interface and storing the clinical trial data related to events along the course of at least one clinical trial on a plurality of nodes of the blockchain data storage system.

In addition, the present invention provides a computer program with program code to perform the method of the present invention when the computer program is executed on a computer.

In context to a blockchain network, nodes are the electronic devices connected to the network and possessing an IP address. Generally, nodes are the communication endpoints which means that any user or application that wants to interact with the blockchain does so through nodes. Therefore, nodes are also a point of communication redistribution. Furthermore, nodes are storage devices, wherein a copy of the blockchain can be stored in each node configured for data storage.

Blockchain works by adding blocks of data and the essence of consensus is to ensure that every block that is added to the chain is the one and only version of the truth that's agreed upon by all the nodes in the system. It is a key feature of the decentralised nature of blockchain.

The base rules for consensus in blockchain include the objective of coming to an agreement; collaboration, cooperation, and equal rights to every node/peer; and mandatory participation of each node in the process.

An example of a consensus mechanism is proof of X (PoX). The idea behind this proof of something (PoX) consensus is to use some scarce resources X where malicious attackers cannot get X easily. In doing so, the system can remain safe in a decentralized and permissionless manner.

In order to gain the privilege of validating transactions, nodes on a PoX network must provide evidence that they have successfully fulfilled X criteria. Oftentimes, this process involves some sort of sacrifice. For example, computational power and effort in proof of work.

An idea of the present invention is thus to provide the advantage that end users gain increased confidence in the accuracy of the information stored along the lifecycle of the clinical trial.

Furthermore, immutable storage and tracking of clinical trial data can be easily ensured with a blockchain data storage system.

According to an aspect of the invention, the events along the course of the at least one clinical trial is at least one of a phase of the clinical trial, a recruitment status of the clinical trial, in particular subjects in recruitment or recruited, formal examination results of the clinical trial, state of the art documents, comparative studies and background data on or for the clinical trial.

Thus, all events along the course of a clinical trial can be safely and immutably stored in the blockchain network.

According to a further aspect of the invention, the background data on or for the clinical trial comprises at least one of implant data, quality of life data, external diagnostics, echocardiograms, x-ray recordings and clinical trial details, in particular a subject name.

Hence, not only experimental data, but also further assisting data such as implant data, if part of the clinical trial, can be stored in the blockchain network. The blockchain network thus enables storage of relevant data from a plurality of sources.

According to a further aspect of the invention, the blockchain data storage system comprises an authorization module configured to grant specific clinical trial members rights to edit specific data elements of the clinical trial data.

Said edited data is then verified by a consensus mechanism when adding new blocks to the chain.

According to a further aspect of the invention, the blockchain data storage system is configured to grant a write permission to a clinical trial contractor and/or a clinical trial subject and a read permission to an auditor and/or observer.

Contractors and/or clinical trials objects are thus advantageously able to fill the blockchain with new clinical trial data whereas auditors and/or observers have a read permission allowing them to access the clinical trial data.

According to a further aspect of the invention, the communication interface is an app of a mobile communication device usable by a clinical trial contractor and/or a clinical trial subject, wherein the communication interface is configured to receive manual data input and/or provides voice-to-text data conversion.

The usage of an app of a mobile communication device advantageously enables an easy and convenient interaction and/or data upload to the blockchain network.

According to a further aspect of the invention, the communication interface is a website configured to receive data input from the clinical trial contractor and/or the clinical trial subj ect.

Alternatively and/or additionally, clinical trial data may be uploaded and/or downloaded to and from the blockchain network through a website communication interface. This provides a further communication interface allowing for additional flexibility to create and edit clinical trial data in the blockchain network.

According to a further aspect of the invention, a separate blockchain is used for each clinical trial or a single blockchain is used for multiple clinical trials.

The blockchain network is thus fully adaptable to the specific requirements of the respective clinical trial and/or contractor requirements. Using a single blockchain for multiple clinical trials provides the advantage of increased efficiency of data storage, whereas using a separate blockchain for each clinical trial provides enhanced security in that once a clinical trial has ended, write permissions may be revoked such that no more blocks can be added to the chain.

According to a further aspect of the invention, the communication interface is configured to receive registration data of the at least one clinical trial, in particular a user ID of a clinical trial subject, and wherein the communication interface is configured to authorize a company and/or person to enter clinical trial data.

The communication interface is thus configured to not only process clinical trial data itself but also metadata of said clinical trial.

According to a further aspect of the invention, the blockchain data storage system is configured to assign an enrolled and/or approved clinical trial to wearable data, a third-party database, weather data and/or a medical device.

Said further data can thus advantageously be linked to clinical trial data in order to expand the clinical trial data set. Said further data may be imported into the blockchain network in order to enhance said clinical trial data.

According to a further aspect of the invention, the blockchain data storage system is configured to assign a clinical trial and/or user ID in response to receiving an instruction from an authorized user.

Assigning said clinical trial and/or user ID can thus be performed by authorized users such as contractors and/or clinical trial subjects.

According to a further aspect of the invention, the communication interface is configured to receive wearable data, in particular generated by a smartring, a smartphone and/or a smartwatch, wherein the wearable data is part of the clinical trial data.

This advantageously provides an increased range of data sources with which the communication interface is capable of exchanging data with in order to expand the data set of said clinical trial.

According to a further aspect of the invention, the blockchain data storage system is configured to encrypt the medical data, in particular by means of a cryptographic hash function. This enables a secure storage of the clinical trial data in the blockchain network.

A cryptographic hash function is an algorithm that takes an arbitrary amount of data and produces a fixed-size output of enciphered text called a hash value.

The herein described features of the blockchain data storage system for clinical trial data are also disclosed for the computer-implemented method for storing clinical trial data on a blockchain data storage system and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a diagram of a blockchain data storage system for clinical trial data according to a preferred embodiment of the invention; and
- Fig. 2: shows a flowchart of a computer-implemented method for storing clinical trial data on a blockchain data storage system according to the preferred embodiment of the invention.

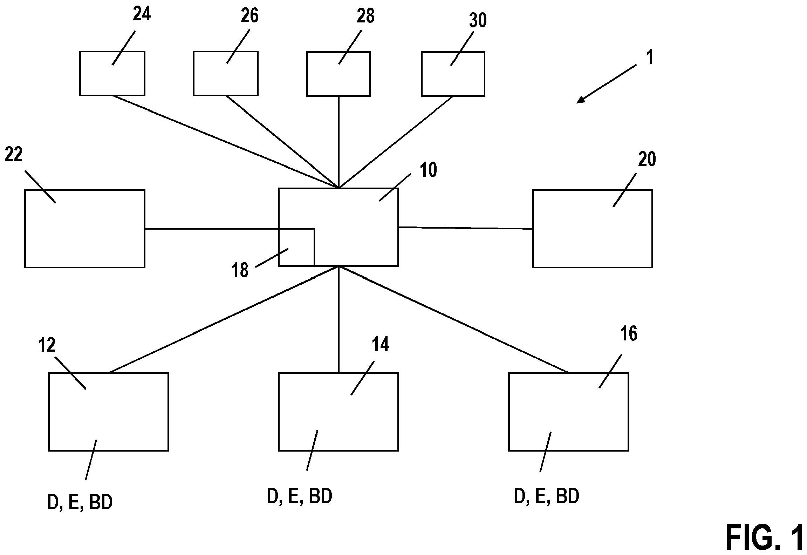

The blockchain data storage system 1 for clinical trial data D shown in Fig. 1 comprises a communication interface 10 configured to receive clinical trial data D and a plurality of nodes 12, 14, 16 configured to store the clinical trial data D related to events E along the course of at least one clinical trial.

The events E along the course of the at least one clinical trial is at least one of a phase of the clinical trial, a recruitment status of the clinical trial, in particular subjects in recruitment or recruited, formal examination results of the clinical trial, state of the art documents, comparative studies and background data BD on or for the clinical trial.

Furthermore, the background data BD on or for the clinical trial comprises at least one of implant data, quality of life data, external diagnostics, echocardiograms, x-ray recordings and clinical trial details, in particular a subject name.

The blockchain data storage system 1 comprises an authorization module 18 configured to grant specific clinical trial members rights to edit specific data elements of the clinical trial data D.

The blockchain data storage system 1 is further configured to grant a write permission to a clinical trial contractor and/or a clinical trial subject and a read permission to an auditor and/or observer.

The communication interface 10 is an app 20 of a mobile communication device usable by a clinical trial contractor and/or a clinical trial subject, wherein the communication interface 10 is configured to receive manual data input and/or provides voice-to-text data conversion.

Alternatively, or in addition, the communication interface 10 can be a website 22 configured to receive data input from the clinical trial contractor and/or the clinical trial subject.

Preferably, a single blockchain is used for multiple clinical trials C. Alternatively, a separate blockchain can be used for each clinical trial.

The communication interface 10 is moreover configured to receive registration data of the at least one clinical trial, in particular a user ID of a clinical trial subject, and wherein the communication interface 10 is configured to authorize a company and/or person to enter clinical trial data D.

The blockchain data storage system 1 is configured to assign an enrolled and/or approved clinical trial to wearable data 24, a third-party database 26, weather data 28 and/or a medical device 30.

In addition, the blockchain data storage system 1 is configured to assign a clinical trial and/or user ID in response to receiving an instruction from an authorized user.

The communication interface 10 is configured to receive wearable data 24, in particular generated by a smartring, a smartphone and/or a smartwatch, wherein the wearable data 24 is part of the clinical trial data D.

Furthermore, the blockchain data storage system 1 is configured to encrypt the medical data, in particular by means of a cryptographic hash function.

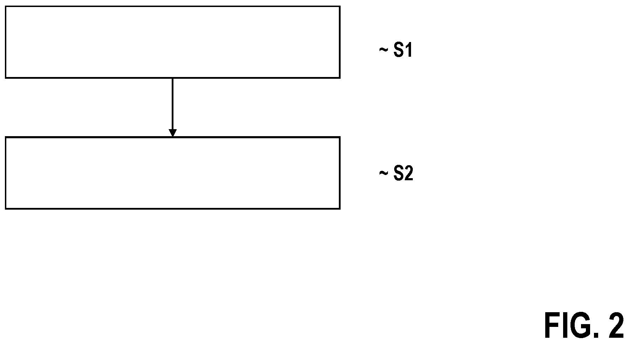

Fig. 2 shows a flowchart of a computer-implemented method for storing clinical trial data D on a blockchain data storage system 1 according to the preferred embodiment of the invention.

The computer-implemented method for storing clinical trial data D on a blockchain data storage system 1 comprises the steps of receiving clinical trial data D by a communication interface 10, and storing the clinical trial data D related to events E along the course of at least one clinical trial on a plurality of nodes 12, 14, 16 of the blockchain data storage system 1.

### Reference Signs

- 1: blockchain data storage system
- 10: communication interface
- 12, 14, 16: nodes
- 18: authorization module
- 20: app
- 22: website
- 24: wearable data
- 26: third-party database
- 28: weather data
- 30: medical device
- BD: background data
- D: clinical trial data
- E: events
- S1 - S2: method steps

## Claims

1. Blockchain data storage system (1) for clinical trial data (D) comprising:
a communication interface (10) configured to receive clinical trial data (D); and
a plurality of nodes (12, 14, 16) configured to store the clinical trial data (D) related to events (E) along the course of at least one clinical trial.

2. Blockchain data storage system of claim 1, wherein the events (E) along the course of the at least one clinical trial is at least one of a phase of the clinical trial, a recruitment status of the clinical trial, in particular subjects in recruitment or recruited, formal examination results of the clinical trial, state of the art documents, comparative studies and background data (BD) on or for the clinical trial.

3. Blockchain data storage system of claim 2, wherein the background data (BD) on or for the clinical trial comprises at least one of implant data, quality of life data, external diagnostics, echocardiograms, x-ray recordings and clinical trial details, in particular a subject name.

4. Blockchain data storage system of any one of the preceding claims, wherein the blockchain data storage system (1) comprises an authorization module (18) configured to grant specific clinical trial members rights to edit specific data elements of the clinical trial data (D).

5. Blockchain data storage system of claim 4, wherein the blockchain data storage system (1) is configured to grant a write permission to a clinical trial contractor and/or a clinical trial subject and a read permission to an auditor and/or observer.

6. Blockchain data storage system of claim 4 or 5, wherein the communication interface (10) is an app (20) of a mobile communication device usable by a clinical trial contractor and/or a clinical trial subject, wherein the communication interface (10) is configured to receive manual data input and/or provides voice-to-text data conversion.

7. Blockchain data storage system of claim 6, wherein the communication interface (10) is a website (22) configured to receive data input from the clinical trial contractor and/or the clinical trial subject.

8. Blockchain data storage system of any one of the preceding claims, wherein a separate blockchain is used for each clinical trial or a single blockchain is used for multiple clinical trials.

9. Blockchain data storage system of any one of the preceding claims, wherein the communication interface (10) is configured to receive registration data of the at least one clinical trial, in particular a user ID of a clinical trial subject, and wherein the communication interface (10) is configured to authorize a company and/or person to enter clinical trial data (D).

10. Blockchain data storage system of any one of the preceding claims, wherein the blockchain data storage system (1) is configured to assign an enrolled and/or approved clinical trial to wearable data (24), a third-party database (26), weather data (28) and/or a medical device (30).

11. Blockchain data storage system of claim 10, wherein the blockchain data storage system (1) is configured to assign a clinical trial and/or user ID in response to receiving an instruction from an authorized user.

12. Blockchain data storage system of claim 10 or 11, wherein the communication interface (10) is configured to receive wearable data (24), in particular generated by a smartring, a smartphone and/or a smartwatch, wherein the wearable data (24) is part of the clinical trial data (D).

13. Blockchain data storage system of any one of the preceding claims, wherein the blockchain data storage system (1) is configured to encrypt the medical data, in particular by means of a cryptographic hash function.

14. Computer-implemented method for storing clinical trial data (D) on a blockchain data storage system (1) comprising the steps of:
receiving (S1) clinical trial data (D) by a communication interface (10); and
storing (S2) the clinical trial data (D) related to events (E) along the course of at least one clinical trial on a plurality of nodes (12, 14, 16) of the blockchain data storage system (1).

15. Computer program with program code to perform the method of claim 14 when the computer program is executed on a computer.
